# EUROPEAN PATENT APPLICATION

(11) **EP 1 977 783 A1**
(43) Date of publication of application: **08.10.2008**
(21) Application number: 08251071.0
(22) Date of filing: 26.03.2008
(51) Int. Cl.: A61M 25/06

(54) **Introducer assembly and method therefor**

(30) Priority: 02.04.2007 US 695438
(71) Applicant: Enpath Medical, Inc., Plymouth, MN 55441 (US)
(72) Inventor: Mauch, Grant A., Delano, MN 55328 (US); Pederson, Brian, East Bethel, Minnesota 55092 (US)
(74) Representative: Lamb, Martin John Carstairs

(57) **Abstract**

An introducer assembly (100) includes a sheath (110) having a sheath proximal end and distal end, and a passage therethrough. The introducer assembly further includes a handle assembly (180) disposed at an angle of about 10-30 degrees relative to the sheath axis. In addition, the introducer assembly includes a dilator (190) having a grip portion, such as a recessed grip portion.

## Description

### Technical Field

Introducers and introducing assemblies, and more specifically an introducer assembly including a sheath assembly and a dilator.

### Background

Introducer devices provide for access to the vascular system and are employed for inserting medical devices such as catheters, guidewires, leads, infusion ports, dialysis ports, dialysis catheters, PICC lines, and others. A typical procedure for gaining access to the central venous system or the arterial system with an introducer is the Seldinger Introduction Method. The Seldinger Method provides for insertion of a needle into the vasculature of a patient. Once the needle is in the vessel, the physician or nurse aspirates the needle or checks for back bleed to assure that the needle is in the vessel, and to draw out air present in the bore of the needle. The syringe is removed and discarded. A guide wire is inserted through the needle, and the needle is removed over the guide wire, leaving the guidewire in the vessel. The introducer, which includes a dilator and the sheath, is placed over the guidewire and inserted into the vessel. For instance, the distal end of the introducer is threaded over the guidewire. With the introducer and wire guide in the vessel, the dilator and wire guide are removed leaving only the sheath in the vessel. The desired medical device is implanted through the passage of the sheath.

The sheath is optionally removed from the medical device, for example by cracking apart the handle, and peeling apart the sheath. However, the initial crack force of cracking apart the handle can be relatively great, rendering the use thereof somewhat awkward or difficult. It is also important to have a dilator that is capable of locking with the sheath. Accordingly, what is needed is an introducer assembly which can be easily removed and further an introducer assembly that allows for the dilator to be effectively coupled with the sheath without inhibiting the use of the introducer.

### Brief Description of the Drawings

- Figure 1: illustrates a perspective view of an introducing assembly as constructed in accordance with at least one embodiment;
- Figure 2A: illustrates a side view of a sheath assembly as constructed in accordance with at least one embodiment;
- Figure 2B: illustrates a perspective view of a sheath assembly as constructed in accordance with at least one embodiment;
- Figure 3: illustrates a perspective view of a portion of a dilator as constructed in accordance with at least one embodiment;
- Figure 4A: illustrates a perspective view of a portion of a dilator as constructed in accordance with at least one embodiment;
- Figure 4B: illustrates a perspective view of a portion of a dilator as constructed in accordance with at least one embodiment;
- Figure 5: illustrates a perspective view of a portion of a dilator as constructed in accordance with at least one embodiment;
- Figure 6: illustrates a top view of an introducing assembly as constructed in accordance with at least one embodiment; and
- Figure 7: illustrates a top view of an introducing assembly as constructed in accordance with at least one embodiment.

### Description of the Embodiments

In the following detailed description, reference is made to the accompanying drawings which form a part hereof, and in which is shown by way of illustration specific embodiments in which the invention may be practiced. These embodiments are described in sufficient detail to enable those skilled in the art to practice the invention, and it is to be understood that other embodiments may be utilized and that structural changes may be made without departing from the scope of the present invention. Therefore, the following detailed description is not to be taken in a limiting sense, and the scope of the present invention is defined by the appended claims and their equivalents.

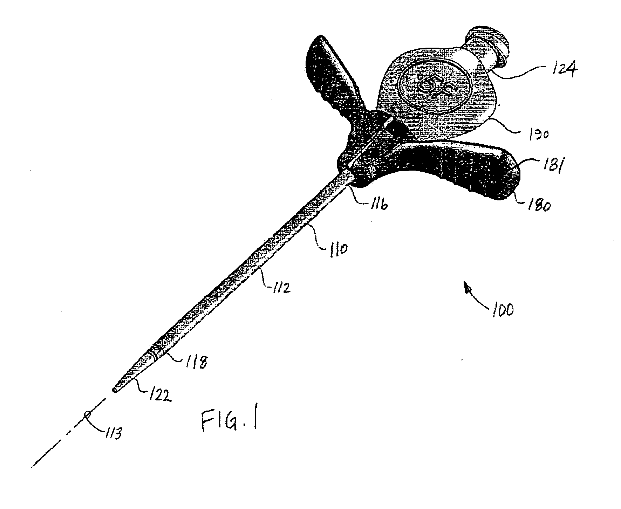

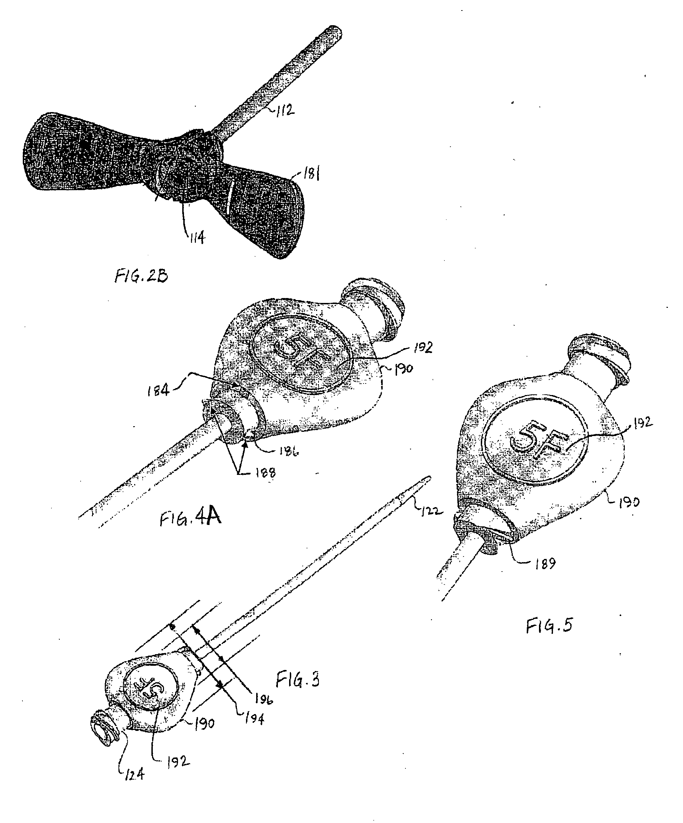

An introducer assembly 100 is illustrated in Figure 1. The introducer assembly 100 includes a dilator 190 and a sheath assembly 110 having a sheath 112 with a passage 114 (Figure 2B) therethrough. The sheath 112 is coupled with a handle assembly 180 as further described below. The sheath 112 extends from a sheath proximal end portion 116 to a sheath distal end portion 118, and is defined in part by a longitudinal axis 113. Near the sheath distal end portion 118, in an option, is a tapered portion, allowing for a more tapered transition portion to taper to the dilator disposed therethrough.

The sheath 112 is formed of, in an example, fluorinated polymers such as, but not limited to, PTFE (PolyTetraFluoroEthylene), FEP (Fluorinated EthylenePropylene), nylon or polyimide. These materials assist in provided lubricious surface proprieties. The sheath material, such as the PTFE, can be molecularly oriented for optionally splitting the sheath. The molecularly oriented sheaths do not necessarily require an additional mechanical scoring operation to produce split lines. Instead, the oriented molecules allow the sheath 112 to naturally peel like a banana.

In a further option, the sheath 112 includes various types of sheaths, for instance, the sheath 112 can comprise a sheath that has a strengthening material, such as a strengthening braid of material. Alternatively, the sheath 112 includes a sheath that is modified to assist in preventing bends and/or kinks along the sheath, or a sheath that is modified to smooth insertion and minimize trauma to a patient.

The introducer assembly 100 further includes an instrument such as a dilator 120 that can be coupled with the sheath assembly 110. The sheath 112 includes a diameter that is sized to receive a dilator 120 therethrough. It should be noted that other instruments such as leads and/or guidewires can be disposed through the sheath and sheath passage, as will further be described below. The dilator 120 extends from a dilator distal end 122 to a dilator proximal end 124, where the dilator distal end is insertable into a patient, for example, over a needle or a guidewire. The dilator distal end 122 optionally ends in a tapered end, allowing for ease of transition within tissue of a patient. The dilator proximal end 124 optionally includes features, such as a luer hub or threads, that allows for other devices to be coupled thereto.

In one embodiment, the handle assembly 180 and the sheath 112 are removable from around instruments disposed therein, such as a lead disposed with the sheath 112. For example, the sheath 112 is removable from around the instrument without having to slide or otherwise manipulate the introducer and/or the sheath over a proximal end of the instrument. In one option, the handle assembly 180 and/or the sheath 112 are removed from an outer perimeter along a cross-section of an instrument disposed therethrough.

The sheath 112 and/or the handle assembly 180, for example, can be removed from the instrument disposed therethrough in a number of different manners. For example, the sheath 112 can include structure integral therewith or non-integral that allows for the sheath 112 to be separated from around the instrument without damaging the instrument, and/or allows for the sheath 112 to be removed from the outer perimeter of the cross-section of the instrument. In some examples, the sheath 112 is coupled with a handle assembly 180, and the handle assembly 180 includes one or more handles 181 that are connected with the sheath 112 to tear the sheath 112 off of the instrument. In another example, the structure includes a tear strip, molecularly orientated material within the sheath, one or more openings in the sheath 112 allowing the sheath 112 to separate at one or more locations that each can be used alone or in combination to separate the sheath 112 from around the instrument. In another option, the sheath 112 is at least partially dissolvable within a body, allowing the sheath 112 to be removed from the instrument. In another option, a slitting or splitting device such as a slitter can be used to removed the sheath 112, where the sheath 112 is removed by slitting. In yet another option, the sheath further includes one, two or more handles which can be used to separate the sheath away from the instrument. Further options include a pre-weakened or scored sheath, allowing for the sheath to be manually removed by tearing, separating, or slitting, for example. In yet another example, the sheath includes molecularly oriented material allowing for the sheath 112 to be removed from around the instrument.

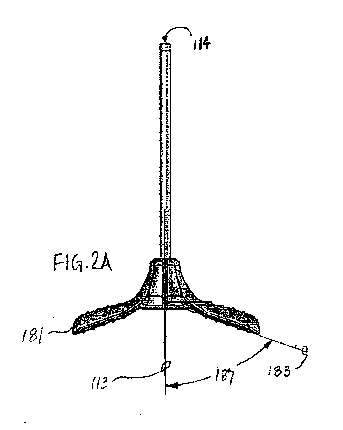

Referring to Figure 2A, the one or more handles 181 is defined in part by a handle longitudinal axis 183. In an option, the handle longitudinal axis 183 is disposed at an angle 187 relative to the sheath longitudinal axis. The angle 187 is measured between the proximal end portion of the sheath and the handle 181, as shown in Figure 2A. In an option, the angle 187 is about 60 - 80 degrees. In another option, the angle 187 is about 70 - 80 degrees. In yet another option, the angle 187 is about 75 degrees.

The introducer assembly 100 optionally includes a valve that is sealingly associated with the passage of the sheath 112, allowing for substantial sealing of the passage 114. The valve assists in preventing fluids to exit from a patient when the sheath 112 is disposed within the patient. While preventing fluids from exiting a patient, the valve permits passage of instruments through the valve, and in an option, substantially seals against the instruments that are disposed therethrough, and assists in preventing air embolism.

The dilator 190 is illustrated in greater detail in Figures 3-5. The dilator 190 includes a gripping portion 192 near a proximal end portion 124 of the assembly 190. The gripping portion 192 includes, in an option, a recessed portion 193. Optionally, one or more indications 195 are provided on the dilator 190, such as at the gripping portion 192, which indicates, for example, the size of the dilator.

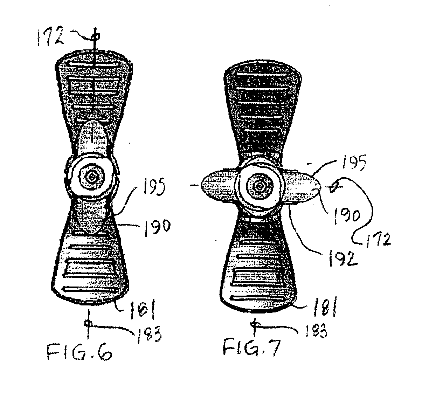

The gripping portion 192 allows for a user to easily grasp the dilator 190 and manipulate the assembly 190. For example, the gripping portion 192 allows for a user to lock the dilator 190, or unlock the dilator 190, or remove the dilator 190 from the outer sheath. In an option, the gripping portion 192 includes an elongate cross-section, for example, an elongate oval cross-section 195, as can be seen in Figures 6 and 7. In another option, the gripping portion 192 has a width greater than about 75 inches. In another option, the gripping portion 192 includes a first width 194 and a second width 196, where the first width 194 is more proximal to a proximal end portion 124 of the dilator 190. The first width 194, in an option, is greater than the second width 196.

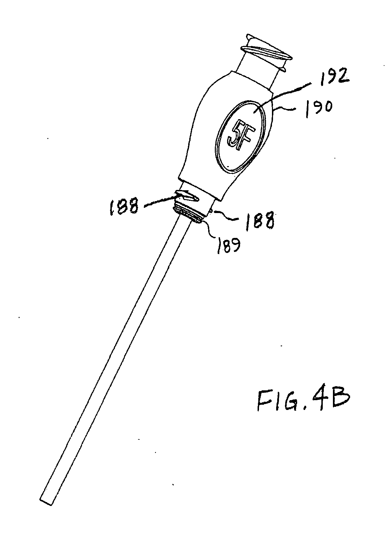

The dilator 190 further includes coupling features associated therewith. For example, the dilator 190 includes an annular snap 189, as shown in Figure 4B. The annular snap 189 can partially or fully surround a perimeter of a portion of the dilator 190. The annular snap 189 mates with corresponding structure of the sheath such that the dilator 190 can be snap-fittedly coupled with the sheath. This can be in addition to or in alternative to other locking features. For instance, in a further example, the dilator 190 includes a locking detent 184, as shown in Figure 4A, and/or a locking feature such as at least one projection 186, as shown in Figures 4A and 4B. The projection 186 includes, but is not limited to, two or more projections 188, as shown in Figures 4A and 4B, or threads such as partial threaded portion 189, as shown in Figure 5. The dilator coupling features are sized and shaped to interact with sheath coupling features (Figure 2B). In an example, as shown in Figure 2B, the sheath assembly 110 includes a locking detent 111 that is adapted to engage locking detent 184 (Figure 4A). In another example, the sheath assembly 110 includes an internal lock 113, such as but not limited to an internal thread that engages the at least one projection 186 and/or the two or more projections 188 (Figure 4A), and/or partial threaded portion 189 (Figure 5).

Referring to Figures 6 and 7, the dilator 190 can be placed in a locked position, such as shown in Figure 6, and an unlocked position, such as shown in Figure 7. In an option, the locked position, the dilator 190 has its gripping portion axis 172 substantially parallel with the handle longitudinal axis 183. In a further option, in an unlocked position, the dilator 190 has its gripping portion axis substantially transverse with the handle longitudinal axis.

In using the introducer assembly, a medical instrument such as a dilator is disposed within a passage of a sheath assembly, the sheath assembly including an elongate introducer sheath extending from a proximal portion to a distal portion, the introducer sheath defined in part by a longitudinal sheath axis, the sheath assembly including a handle assembly having one or more handles, the one or more handles defined in part by a longitudinal handle axis, the dilator including a dilator hub, the dilator hub having a grip portion, the grip portion having an optional elongate cross-section. In another option, the longitudinal handle axis is disposed at an angle of about 10-30 degrees relative to the longitudinal sheath axis. The dilator is gripped at the dilator grip portion, for example at a recess within the dilator grip portion and optionally removed from the sheath assembly.

In a further option, the dilator is locked with the sheath assembly, where optionally locking the dilator includes disposing a dilator gripping portion axis substantially parallel with the longitudinal handle axis, and/or locking the dilator includes rotating the dilator relative to the sheath assembly. In another option, unlocking the dilator from the sheath assembly, including disposing a dilator gripping portion axis substantially transverse to the longitudinal handle axis.

The introducer assembly is passed over a guidewire into a cavity or a vessel. The dilator is moved and the sheath is used to introduce PICC liens, catheters, leads or other diagnostic or therapeutic devices. After a diagnostic and/or therapeutic device is inserted through the sheath assembly, the sheath assembly is removed, for example, by splitting the sheath assembly, and the diagnostic or therapeutic device remains through the skin of the patient.

Advantageously, the introducer assembly described above provides many benefits. For example, dilator allows for a larger gripping portion, and a larger flat area to improve torqueability. The larger size further allows for the application of a relatively large size to be indicated thereon. Still further, the dilator gripping portion allows for the locking orientation to provide a visual and/ot tactile indication of locked or unlocked status. The introducer assembly further allows for removal of the introducer without disruption to the procedure or placement of the medical device such as a lead.

It is to be understood that the above description is intended to be illustrative, and not restrictive. Many other embodiments will be apparent to those of skill in the art upon reading and understanding the above description. It should be noted that embodiments or portions thereof discussed in different portions of the description or referred to in different drawings can be combined to form additional embodiments of the present invention. The scope of the invention should, therefore, be determined with reference to the appended claims, along with the full scope of equivalents to which such claims are entitled.

## Claims

1. An introducer assembly comprising:
an elongate introducer sheath extending from a proximal portion to a distal portion, the introducer sheath defined in part by a longitudinal sheath axis, the elongate introducer sheath including a passage therethrough;
a handle assembly coupled with the proximal portion of the introducer sheath, the handle assembly including one or more handles;
the one or more handles defined in part by a longitudinal handle axis; and
the longitudinal handle axis is disposed at an angle of about 10-30 degrees relative to the longitudinal sheath axis.

2. The introducer assembly as recited in claim 1, further comprising a dilator, the dilator extending from a dilator proximal portion to a dilator distal portion, the dilator including a dilator grip.

3. The introducer assembly as recited in claim 2, wherein the dilator grip has an elongate cross-section defined in part by a grip longitudinal axis.

4. The introducer assembly as recited in claim 3, wherein the dilator has a locked position and an unlocked position, wherein the grip longitudinal axis is substantially parallel with the longitudinal handle axis when the dilator is in the locked position.

5. The introducer assembly as recited in claim 3, wherein the dilator has a locked position and an unlocked position, wherein the grip longitudinal axis is substantially transverse to the longitudinal handle axis when the dilator is in the unlocked position.

6. The introducer assembly as recited in claim 2, wherein the dilator grip includes a recessed portion.

7. The introducer assembly as recited in claim 2, wherein the dilator grip includes at least one indication thereon.

8. The introducer assembly as recited in claim 1, wherein the dilator includes an annular snap.

9. An introducer assembly comprising:
an elongate introducer sheath extending from a proximal portion to a distal portion, the introducer sheath defined in part by a longitudinal sheath axis, the elongate introducer sheath including a passage therethrough;
a handle assembly coupled with the proximal portion of the introducer sheath, the handle assembly including one or more handles, the one or more handles defined in part by a longitudinal handle axis; and
a dilator including a dilator hub, the dilator hub having a grip portion, the grip portion having an elongate cross-section.

10. The introducer assembly as recited in claim 9, wherein the longitudinal handle axis is disposed at an angle of about 10-30 degrees relative to the longitudinal sheath axis.

11. The introducer assembly as recited in claim 9, wherein the dilator grip portion has an elongate oval cross-section.

12. The introducer assembly as recited in claim 9, wherein the grip portion having a width greater than about .75 inches.

13. The introducer assembly as recited in claim 9, wherein the grip portion has a first width and a second width, and the first width is greater than the second width, the first width is more proximal to a proximal end portion of the dilator.

14. The introducer assembly as recited in claim 9, wherein the grip portion includes at least one recessed portion.

15. A method comprising:
disposing a dilator within a passage of a sheath assembly, the sheath assembly including an elongate introducer sheath extending from a proximal portion to a distal portion, the introducer sheath defined in part by a longitudinal sheath axis, the sheath assembly including a handle assembly having one or more handles, the one or more handles defined in part by a longitudinal handle axis, the longitudinal handle axis is disposed at an angle of about 10-30 degrees relative to the longitudinal sheath axis; and
gripping the dilator at a recessed dilator gripping portion.

16. The method as recited in claim 15, further comprising locking the dilator with the sheath assembly.

17. The method as recited in claim 16, wherein locking the dilator includes disposing a dilator gripping portion axis substantially parallel with the longitudinal handle axis.

18. The method as recited in claim 16, wherein locking the dilator includes rotating the dilator relative to the sheath assembly.

19. The method as recited in claim 15, further comprising snap-fittedly coupling the dilator with the sheath.

20. The method as recited in claim 15, further comprising removing the sheath assembly from the dilator with the handle assembly.

21. A method comprising:
disposing a dilator within a passage of a sheath assembly, the sheath assembly including an elongate introducer sheath extending from a proximal portion to a distal portion, the introducer sheath defined in part by a longitudinal sheath axis, the sheath assembly including a handle assembly having one or more handles, the one or more handles defined in part by a longitudinal handle axis, the dilator including a dilator hub, the dilator hub having a grip portion, the grip portion having an elongate cross-section; and
gripping the dilator at the dilator grip portion.

22. The method as recited in claim 21, wherein gripping the dilator includes gripping a recess within the dilator grip portion.

23. The method as recited in claim 21, further comprising locking the dilator with the sheath assembly.

24. The method as recited in claim 23, wherein locking the dilator includes disposing a dilator gripping portion axis substantially parallel with the longitudinal handle axis.

25. The method as recited in claim 23, wherein locking the dilator includes rotating the dilator relative to the sheath assembly.

26. The method as recited in claim 21, further comprising unlocking the dilator from the sheath assembly, including disposing a dilator gripping portion axis substantially transverse to the longitudinal handle axis.
